(19) Europäisches Patentamt
European Patent Office
Office européen des brevets

(11) **EP 2 659 886 A1**

(12) **EUROPEAN PATENT APPLICATION**
published in accordance with Art. 153(4) EPC

(43) Date of publication:
06.11.2013 Bulletin 2013/45

(51) Int Cl.:
*A61K 31/165* (2006.01)     *A61K 9/70* (2006.01)
*A61K 47/10* (2006.01)     *A61K 47/14* (2006.01)
*A61K 47/32* (2006.01)     *A61P 13/02* (2006.01)

(21) Application number: 10861516.2

(22) Date of filing: 28.12.2010

(86) International application number:
PCT/JP2010/073783

(87) International publication number:
WO 2012/090322 (05.07.2012 Gazette 2012/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(71) Applicant: **Sekisui Medical Co., Ltd.**
**Tokyo 103-0027 (JP)**

(72) Inventors:
• **UDAGAWA Hiroko**
**Amagasaki-shi**
**Hyogo 661-8564 (JP)**

• **KOMODA Toshikazu**
**Amagasaki-shi**
**Hyogo 661-8564 (JP)**
• **SHIBATA Sakiko**
**Tokyo 103-0027 (JP)**
• **HAMABE Masaru**
**Tokyo 103-0027 (JP)**

(74) Representative: **Ter Meer Steinmeister & Partner**
**Me/bk**
**Mauerkircherstrasse 45**
**81679 München (DE)**

(54)     **PATCH**

(57)     Disclosed is a patch excellent in storage stability and transdermal absorbability of midodrine and having good adhesion. The patch includes a backing and a medicated layer stacked on one surface of the backing so as to be integrated with the backing. The medicated layer contains 3 to 40% by weight of midodrine and 60 to 97% by weight of an acrylic adhesive including a copolymer formed by copolymerization of monomers including 60 to 98% by weight of an alkyl acrylate having an alkyl group with 1 to 12 carbon atoms and 2 to 40% by weight of 1-vinyl-2-pyrrolidone. In the medicated layer, part of midodrine is present in an undissolved state.

Fig. 7

EP 2 659 886 A1

**Description**

Field

The present invention relates to a patch for transdermal administration of midodrine.

Background

Midodrine is known to increase blood pressure because its active metabolite, 2-amino-1-(2',5'-dimethoxyphenyl) ethanol (hereinafter referred to as "DMAE"), selectively stimulates $\alpha_1$-receptors to constrict peripheral blood vessels, and is therefore used for treatment of essential hypotension and orthostatic hypotension. In clinical sites, the smooth muscle contractile effect of DMAE through stimulation of $\alpha_1$-receptors is recently receiving attention, and it is expected to apply midodrine and DMAE to treatment of stress urinary incontinence.

One currently commercially available medicine containing midodrine as its component is an oral medicine containing midodrine hydrochloride as a principal ingredient, and 2 to 10 mg of the oral medicine is taken 2 or 3 times a day, according to its general dosage and administration. However, after the administration of the oral medicine, a rapid increase in the blood level of DMAE, which is the metabolite of midodrine hydrochloride, occurs in some cases, and this may cause supine hypertension as a side effect.

One method proposed to solve the above problem is to apply a patch containing midodrine to skin to allow the midodrine to be transdermally administered (Patent Literature 1). The advantages of the administration of midodrine using such a patch are: (1) Since midodrine can be absorbed through the skin at a slow rate for a long period of time, a rapid increase in the blood level of DMAE is less likely to occur. (2) Primary metabolism of midodrine in the liver is avoided, and bioavailability is improved. (3) Even when a side effect occurs, the administration of midodrine can be immediately stopped by removing the patch.

Particularly, patients with stress urinary incontinence essentially do not require blood pressure control. Therefore, when midodrine is used for the treatment of stress urinary incontinence, the use of a patch is preferable because midodrine can be easily administered to such patients continuously in an amount that poses only a small possibility of causing hypertension as a side effect.

As described above, Patent Literature 1 proposes the use of a transdermal delivery formulation containing midodrine as a patch and also proposes a transdermal delivery formulation containing a polar solvent, an absorption promoter, and a basic substance as additives for improving the transdermal absorbability of midodrine. The proposed forms of the transdermal delivery formulation include a patch, an ointment, a cream, and a lotion.

However, the present inventors have conducted studies and found that the additives used in the above transdermal delivery formulation are not always suitable for the additives for a patch. More specifically, the inventors have found a problem in that satisfactory transdermal absorbability of midodrine is not achieved because these additives are not favorably dissolved in an adhesive used as the base of the patch and because deterioration of the base and decomposition of midodrine occur during long-term storage.

In addition, a midodrine patch is required to allow midodrine to be transdermally absorbed such that the midodrine is converted to DMAE in the body and that its blood level is increased to a desired range and is maintained for the required period of time.

To achieve such an effect, it is necessary that a sufficient amount of midodrine be contained in a medicated layer and that the midodrine be continuously supplied to the surface applied to the skin so as to be transferred to the skin at a desired rate.

To achieve such a duration of the transdermal absorbability of midodrine, the present inventors have conducted studies on various bases and additives. When a base having a high ability to dissolve midodrine and allowing midodrine to easily diffuse therein is selected, the releasability of midodrine from the medicated layer and the transferability of midodrine to the skin are not maintained, so that the desired transdermal absorption rate of midodrine cannot be ensured for a prescribed period of time.

In contrast, when a base having a low ability to dissolve midodrine is selected, part of midodrine contained in the medicated layer will be present as crystals. In this case, the midodrine present in an undissolved state in the medicated layer often cannot dissolve and diffuse rapidly. Therefore, midodrine is not supplied continuously to the skin surface, so that the desired transdermal absorption rate of midodrine cannot be maintained. In addition, the deposition state of the crystals of midodrine may be changed during storage of the patch, so that the transdermal absorbability of midodrine is affected.

Citation List

Patent Literature

Patent Literature 1: Japanese Patent Application Laid-Open No. 2001-131062

Summary

Technical Problem

The present invention provides a patch excellent in storage stability and transdermal absorbability of midodrine and having good adhesion.

Solution to Problem

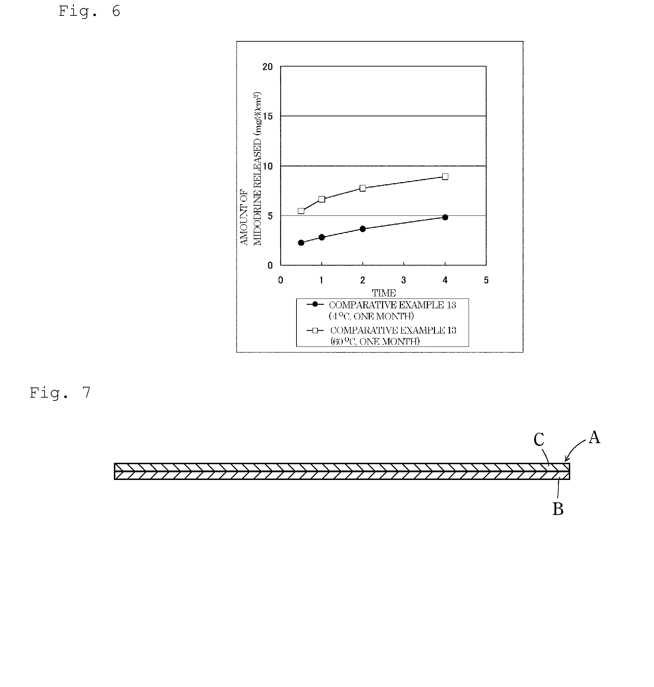

The patch A of the present invention includes a backing B and a medicated layer C stacked on one surface of the backing B so as to be integrated with the backing B, as shown in FIG. 7. The medicated layer C contains 3 to 40% by weight of midodrine and 60 to 97% by weight of an acrylic adhesive including a copolymer prepared by copolymerization of monomers including 60 to 98% by weight of an alkyl acrylate having an alkyl group with 1 to 12 carbon atoms and 2 to 40% by weight of 1-vinyl-2-pyrrolidone. In the medicated layer C, part of the midodrine is present in an undissolved state.
The type of backing constituting the patch of the present invention is not particularly limited. Preferably, the backing can prevent loss of midodrine in the medicated layer, can protect the medicated layer, has strength sufficient to provide self-supportability to the patch, and also has flexibility sufficient to provide favorable application feeling to the patch.
The type of backing to be used is not particularly limited, and examples thereof may include a non-foamed resin sheet, a foamed resin sheet, a nonwoven fabric, a woven fabric, a knitted fabric, and an aluminum sheet. The backing may be composed of a single layer or a plurality of stacked and integrated layers.
Examples of the resin constituting the above non-foamed or foamed resin sheet may include cellulose acetate, ethyl cellulose, rayon, polyethylene terephthalate, plasticized vinyl acetate-vinyl chloride copolymers, nylon, ethylene-vinyl acetate copolymers, plasticized polyvinyl chloride, polyurethane, polyethylene, polypropylene, and polyvinylidene chloride. Of these, polyethylene terephthalate is preferred.
Preferably, the backing is prepared by stacking a polyethylene terephthalate film on a nonwoven fabric or a resin sheet and integrating them with each other, from the viewpoint of the flexibility of the backing and its effect of preventing loss of midodrine. Examples of the material constituting the nonwoven fabric or the resin sheet may include polyethylene, polypropylene, ethylene-vinyl acetate copolymers, ethylene-methyl (meth)acrylate copolymers, nylon, polyester, vinylon, SIS copolymers, SEBS copolymers, rayon, and cotton. These materials may be used alone or in combination of two or more.
In the case in which the backing is prepared by stacking and integrating a polyethylene terephthalate film and a nonwoven fabric, if the thickness of the polyethylene terephthalate film is small, the polyethylene terephthalate film may not be uniformly bonded to the nonwoven fabric when these are stacked and integrated, or the strength of the obtained backing may become insufficient. In addition, pinholes may be formed, so that peeling is likely to occur at the interface between the polyethylene terephthalate film and the nonwoven fabric. If the thickness of the polyethylene terephthalate film is large, the flexibility of the obtained backing may become insufficient, and the conformability of the patch to the skin when the patch is applied may deteriorate, so that the application feeling of the patch may deteriorate. Therefore, the thickness of the polyethylene terephthalate film is preferably 2 to 50 $\mu$m and more preferably 2 to 25 $\mu$m.
In the case in which the backing is prepared by stacking and integrating the polyethylene terephthalate film and the nonwoven fabric, if the basis weight of the nonwoven fabric is small, the adhesion between the nonwoven fabric and the polyethylene terephthalate film may become deteriorated, or the strength of the backing may become insufficient. If the basis weight of the nonwoven fabric is large, the flexibility of the backing may become low. Therefore, the basis weight of the nonwoven fabric is preferably 10 to 300 g/m$^2$.
The type of method of producing the backing by stacking and integrating the polyethylene terephthalate film and the nonwoven fabric is not particularly limited, and examples of the production method may include a method in which these are stacked and integrated using a binder and a method using heat fusion. The strength and texture of the backing can be controlled during the production of the backing by adding a binder or partially heat-fusing the polyethylene terephthalate film and the nonwoven fabric.
The medicated layer is stacked on one surface of the backing to be integrated with the backing. This medicated layer contains an acrylic adhesive and midodrine.
The acrylic adhesive includes a copolymer prepared by copolymerization of monomers including 60 to 98% by weight of an alkyl acrylate having an alkyl group with 1 to 12 carbon atoms and 2 to 40% by weight of 1-vinyl-2-pyrrolidone.

Since the acrylic adhesive contains the above copolymer, midodrine can be stably and favorably dissolved in the medicated layer, and undissolved midodrine in the medicated layer is allowed to rapidly dissolve in the medicated layer as the midodrine dissolved in the medicated layer is absorbed into the skin.

The type of alkyl acrylate having an alkyl group with 1 to 12 carbon atoms is not particularly limited, and examples thereof may include methyl acrylate, ethyl acrylate, propyl acrylate, butyl acrylate, hexyl acrylate, heptyl acrylate, octyl acrylate, 2-ethylhexyl acrylate, nonyl acrylate, decyl acrylate, undecyl acrylate, and dodecyl acrylate.

The amount of the alkyl acrylate having an alkyl group with 1 to 12 carbon atoms in the copolymer is limited to 60 to 98% by weight and is preferably 70 to 95% by weight.

If the amount of the alkyl acrylate having an alkyl group with 1 to 12 carbon atoms in the copolymer is small, the dissolvability of midodrine in the medicated layer becomes very high, and this causes a problem in that midodrine is not delivered to the skin and remains in the medicated layer. In one case, the medicated layer becomes hard, so that the adhesion of the medicated layer to the skin is lowered. In another case, the hydrophilicity of the acrylic adhesive becomes excessively high, and the acrylic adhesive absorbs moisture during sweating. These cause a reduction in the adhesion of the acrylic adhesive, so that the patch may be easily peeled off.

If the amount of the alkyl acrylate having an alkyl group with 1 to 12 carbon atoms in the copolymer is large, the dissolvability of midodrine in the medicated layer becomes small. Therefore, after the patch is applied to the skin, the desired absorption rate of midodrine cannot be achieved.

Preferably, the alkyl acrylate having an alkyl group with 1 to 12 carbon atoms includes an alkyl acrylate having an alkyl group with 1 to 3 carbon atoms and an alkyl acrylate having an alkyl group with 4 to 12 carbon atoms.

Examples of the alkyl acrylate having an alkyl group with 1 to 3 carbon atoms may include methyl acrylate, ethyl acrylate, and propyl acrylate, and ethyl acrylate is preferred because the acrylic adhesive has a favorable diffusibility of midodrine and adhesion.

Examples of the alkyl acrylate having an alkyl group with 4 to 12 carbon atoms may include butyl acrylate, hexyl acrylate, heptyl acrylate, octyl acrylate, 2-ethylhexyl acrylate, nonyl acrylate, decyl acrylate, undecyl acrylate, and dodecyl acrylate. The copolymer contains preferably an alkyl acrylate having an alkyl group with 4 to 8 carbon atoms, more preferably any of butyl acrylate, hexyl acrylate, octyl acrylate, and 2-ethylhexyl acrylate, and particularly preferably any of n-octyl acrylate and 2-ethylhexyl acrylate, because the adhesion of the acrylic adhesive can be well balanced with the dissolvability of midodrine therein.

When the acrylic adhesive contains the alkyl acrylate component having an alkyl group with 1 to 3 carbon atoms, the alkyl acrylate component having an alkyl group with 4 to 12 carbon atoms, and a 1-vinyl-2-pyrrolidone component, these components are preferably as follows.

The amount of the alkyl acrylate component having an alkyl group with 1 to 3 carbon atoms is preferably 25 to 70% by weight and more preferably 30 to 60% by weight. The amount of the alkyl acrylate component having an alkyl group with 4 to 12 carbon atoms is preferably 25 to 70% by weight, more preferably 30 to 60% by weight, and particularly preferably 30 to 45% by weight. The amount of the 1-vinyl-2-pyrrolidone component is preferably 2 to 30% by weight and more preferably 5 to 20% by weight.

If the amount of the alkyl acrylate having an alkyl group with 1 to 3 carbon atoms is small, the rate of dissolution of midodrine in the medicated layer and the diffusibility of midodrine in the acrylic adhesive may become small, so that it may be difficult to maintain sustained release of midodrine from the patch.

If the amount of the alkyl acrylate having an alkyl group with 1 to 3 carbon atoms is large, the adhesion of the acrylic adhesive to the skin becomes low, and the reliability of the administration of the drug may become low.

If the amount of the alkyl acrylate having an alkyl group with 4 to 12 carbon atoms is small, the flexibility of the acrylic adhesive becomes insufficient, and the adhesion of the patch to the skin becomes low, so that the patch may be easily peeled off from the skin.

If the amount of the alkyl acrylate having an alkyl group with 4 to 12 carbon atoms is large, the polarity of the acrylic adhesive becomes low, and the dissolvability of midodrine in the medicated layer becomes low. In addition, the cohesion of the acrylic adhesive increases, and the diffusibility of midodrine in the acrylic adhesive becomes low. Therefore, it is difficult to maintain high releasability of midodrine in order to allow a constant amount of midodrine to be administered for a long period of time.

The copolymer included in the acrylic adhesive contains the 1-vinyl-2-pyrrolidone component. In contrast to methacrylic acid and acrylic acid commonly used as a constituent monomer for imparting cohesion to an adhesive, 1-vinyl-2-pyrrolidone can impart cohesion but does not have an acid at its terminals. Therefore, 1-vinyl-2-pyrrolidone does not react with a basic drug such as midodrine and causes less skin irritation.

The amount of 1-vinyl 2-pyrrolidone in the copolymer constituting the acrylic adhesive is limited to 2 to 40% by weight and is preferably 2 to 30% by weight and more preferably 5 to 20% by weight.

If the amount of 1-vinyl-2-pyrrolidone in the copolymer is small, the dissolvability of midodrine in the medicated layer becomes low, so that the desired absorption rate of midodrine cannot be achieved after the patch is applied to the skin.

If the amount of 1-vinyl-2-pyrrolidone in the copolymer is large, the dissolvability of midodrine in the medicated layer becomes very high, and this causes a problem that midodrine is not delivered to the skin and remains in the medicated layer. In one case, the medicated layer becomes hard, so that the adhesion of the medicated layer to the skin is lowered. In another case, the hydrophilicity of the acrylic adhesive becomes excessively high, and the acrylic adhesive absorbs moisture during sweating. These cause a reduction in the adhesion of the acrylic adhesive, so that the patch may be easily peeled off.

The acrylic adhesive may contain an additional monomer component, so long as the storage stability and transdermal absorbability of midodrine and the adhesion to the skin are not impaired.

Examples of the additional monomer component may include: alkyl methacrylates having an alkyl group with 1 to 12 carbon atoms such as methyl methacrylate, ethyl methacrylate, butyl methacrylate, 2-ethylhexyl methacrylate, and dodecyl methacrylate; polyfunctional monomers having at least two polymerizable functional groups in their molecule such as divinylbenzene, methylene bisacrylic acid, ethylene glycol dimethacrylate, and ethylene glycol diacrylate; acrylic acid; methacrylic acid; vinylpyrrolidone; vinyl acetate; and hydroxyalkyl (meth)acrylates. By adding such a monomer component to the acrylic adhesive, the dissolvability and diffusibility of midodrine in the medicated layer and the adhesion of the medicated layer of the patch can be controlled.

In addition to the above polyfunctional monomers, a cross-linking agent such as an epoxy compound, a polyisocyanate compound, a metal chelate compound, or a metal alkoxide compound may be further added to the acrylic adhesive, so long as the storage stability of midodrine is not impaired.

When a cross-linking agent is added to the monomers used as the raw materials of the acrylic adhesive, the internal cohesion of the acrylic adhesive increases, and therefore the adhesive is less likely to remain on the skin when the patch is removed from the skin. The amount of the cross-linking agent added is preferably 0.01 to 3 parts by weight based on 100 parts by weight of the monomers used as the raw materials of the acrylic adhesive.

The amount of the acrylic adhesive in the medicated layer is limited to 60 to 97% by weight and is preferably 65 to 90% by weight and more preferably 70 to 85% by weight.

If the amount of the acrylic adhesive in the medicated layer is small, the relative amount of midodrine in the medicated layer becomes large, and therefore the absorption rate of midodrine is reduced, so that midodrine in the medicated layer is wasted. In addition, the amount of crystals of midodrine deposited in the medicated layer becomes excessively large, so that the adhesion of the patch becomes low.

If the amount of the acrylic adhesive in the medicated layer is large, the relative amount of midodrine in the medicated layer becomes small, and the amount of transdermal absorption of midodrine becomes small. Therefore, the blood level of DMAE cannot be increased to the desired range unless the application area of the patch to the skin is increased.

Any conventionally known method may be used as the polymerization method for the acrylic adhesive. For example, the above-described monomers are mixed in the presence of a polymerization initiator and then polymerized by solution polymerization. More specifically, prescribed amounts of the monomers, the polymerization initiator, an optionally added cross-linking agent, and a solvent are supplied to a reaction vessel equipped with a stirrer and a cooling and refluxing device for the vaporized solvent. The mixture is heated at a temperature of 60 to 80°C in preferably a nitrogen gas atmosphere for 4 to 48 hours to subject the monomers to a radical polymerization reaction. These monomers and solvent may be added simultaneously or in several times, as appropriate, to the reaction vessel.

Examples of the polymerization initiator may include: azobis-based polymerization initiators such as 2,2'-azobisisobutyronitrile (AIBN), 1,1'-azobis(cyclohexane-1-carbonitrile), and 2,2'-azobis-(2,4'-dimethylvaleronitrile); and peroxide-based polymerization initiators such as benzoyl peroxide (BPO), lauroyl peroxide (LPO), and di-tert-butyl peroxide. Examples of the solvent include ethyl acetate and toluene.

The styrene-equivalent weight-average molecular weight of the acrylic adhesive measured by Gel Permeation Chromatography (GPC) is preferably 100,000 to 3,000,000.

The acrylic adhesive constituting the medicated layer contains midodrine. The midodrine used in the present invention is of the free-base type and does not include salt-added type midodrine such as midodrine hydrochloride.

The amount of midodrine in the medicated layer is limited to 3 to 40% by weight and is preferably 5 to 25% by weight and more preferably 10 to 20% by weight.

If the amount of midodrine in the medicated layer is small, the amount of transdermal absorption of midodrine becomes small. Therefore, the blood level of DMAE cannot be increased to the desired level unless the application area of the patch to the skin is increased.

If the amount of midodrine in the medicated layer is large, the absorption rate of midodrine becomes low, so that midodrine in the medicated layer is wasted. In addition, the amount of crystals of midodrine deposited in the medicated layer becomes excessively large, so that the adhesion of the patch becomes low.

Part of midodrine is dissolved in the medicated layer, but the other part of midodrine is present in an undissolved state in the medicated layer. As described above, since part of midodrine present in the medicated layer is not dissolved therein, the patch allows the transdermal absorbability of midodrine to be maintained stably for a long period of time.

A method of determining whether or not there is midodrine undissolved in the medicated layer will next be described. First, polyethylene terephthalate films having a thickness of 25 $\mu$m and a haze value of 2% are applied to the both faces of a measurement medicated layer used as a measurement target. The haze value is measured at three arbitrary points on the measurement medicated layer using a haze meter. The arithmetic mean of the haze values at the three points is used as the haze value of the measurement medicated layer. For example, a commercially available device available under product name "300A" from Nippon Denshoku Industries Co., Ltd. can be used as the haze meter. Examples of the polyethylene terephthalate film having a thickness of 25 $\mu$m and a haze value of 2% include a commercially available polyethylene terephthalate film available under product name "Teijin Tetoron film" from Teijin DuPont Films Japan Limited.

Next, a medicated layer having the same composition as that of the measurement medicated layer except that it contains no midodrine and medicated layers having the same composition as that of the measurement medicated layer except that they contain different amounts (% by weight) of midodrine are prepared as reference medicated layers. The haze values of these reference medicated layers are measured using the above-described procedure.

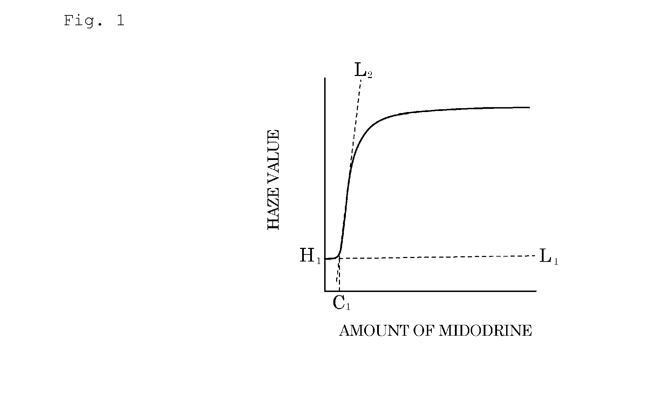

A graph is drawn with the obtained haze values on the vertical axis and the amounts (% by weight) of midodrine in the medicated layer on the horizontal axis. This graph is shown in FIG. 1. As can be seen from the graph in FIG. 1, the haze value of the medicated layer increases steeply when the amount (% by weight) of midodrine is equal to or larger than a given amount. This increase in haze value is due to undissolved midodrine present in the medicated layer.

A first calibration line $L_1$ is drawn on the graph shown in FIG. 1 on the basis of the measurement values before the steep increase in haze value. In addition, a second calibration line $L_2$ is drawn on the graph shown in FIG. 1 on the basis of the measurement values immediately after the steep increase in haze value.

The amount $C_1$ (% by weight) of midodrine at the intersection of the first calibration line $L_1$ and the second calibration line $L_2$ is the saturating concentration of midodrine in the acrylic adhesive in the medicated layer. The haze value $H_1$ of the medicated layer at the intersection of the first calibration line $L_1$ and the second calibration line $L_2$ is the haze value of the medicated layer in which midodrine is dissolved in the medicated layer at the saturating concentration.

Therefore, when the haze value of the measurement medicated layer is equal to or lower than $H_1$, it can be determined that there is no midodrine present in an undissolved state in the measurement medicated layer. And, when the haze value of the measurement medicated layer exceeds $H_1$, it can be determined that there is midodrine present in an undissolved state in the measurement medicated layer.

The total amount of midodrine in a medicated layer can be measured by the following procedure. First, a test piece having an area of 3 cm$^2$ is cut from a patch, and the weight $W_1$ (g) of the test piece is measured.

Then the test piece is immersed in 5 mL of a first solvent mixture composed of 80% by volume of ethyl acetate and 20% by volume of ethanol to dissolve the medicated layer. Next, a second solvent mixture composed of 60% by volume of water and 40% by volume of acetonitrile is added to the first solvent mixture to prepare an extract solution, and the acrylic adhesive is allowed to coagulate in the extract solution.

Next, a supernatant obtained by centrifugation of the extract solution is subjected to HPLC measurement at a wavelength of 220 nm using octadecyl-silylated silica gel ($\phi$4.6 mm × 150 mm) to measure the amount $W_2$ (g) of midodrine in the medicated layer of the test piece.

The backing is removed from the extract solution, washed, and dried, and then the weight $W_3$ (g) of the backing is measured.

Then the amount $C_2$ (% by weight) of undissolved midodrine present in the medicated layer can be computed from the following formula using the total weight $W_4$ (g) of the entire medicated layer obtained by subtracting the weight $W_3$ (g) of the backing from the weight $W_1$ (g) of the test piece, the amount $W_2$ (g) of midodrine, and the saturating concentration $C_1$ (% by weight) of midodrine.

```
Amount of midodrine (% by weight) C₂

   = [W₂/W₄ - ((W₄ - W₂) × C₁/(100 - C₁))/W₄] × 100
```

The amount (% by weight) of midodrine in each reference medicated layer prepared for obtaining the saturating concentration $C_1$ of midodrine can be determined from the following formula after the procedure same as that described above is performed.

```
Amount of midodrine (% by weight) = W₂ × 100/W₄
```

In the medicated layer, the amount of undissolved midodrine in the medicated layer is preferably 40 to 97% by weight, more preferably 50 to 97% by weight, and particularly preferably 80 to 95% by weight based on the total amount of midodrine contained in the medicated layer.

In the medicated layer, if the amount of undissolved midodrine in the medicated layer is small, the duration of transdermal absorbability of midodrine cannot be maintained. In the medicated layer, if the amount of undissolved midodrine in the medicated layer is large, the transdermal absorbability of midodrine immediately after the patch is applied to the skin becomes low.

The patch of the present invention is used with its medicated layer applied to the skin, and midodrine contained in the medicated layer is absorbed through the skin continuously, so that high transdermal absorbability is achieved. To further improve the transdermal absorbability, it is preferable to improve the dissolution rate and the diffusion rate of the midodrine present in an undissolved state in the medicated layer into the medicated layer.

When a liquid solubilizing aid is further added to the medicated layer, the midodrine present in the undissolved state in the medicated layer can be smoothly dissolved and diffused in the medicated layer as the amount of midodrine in the acrylic adhesive decreases. Therefore, the ability to supply midodrine in the medicated layer to the skin is enhanced, so that the duration of the transdermal absorbability of midodrine can be further improved.

The liquid solubilizing aid must be liquid under the conditions of 1 atm ($1.01 \times 10^5$ Pa) and 20°C. The liquid solubilizing aid is preferably a monohydric alcohol or a fatty acid ester obtained by dehydration condensation of a fatty acid and an aliphatic alcohol. Examples of the fatty acid may include lauric acid, myristic acid, palmitic acid, stearic acid, oleic acid, sebacic acid, and adipic acid. Examples of the aliphatic alcohol may include methanol, ethanol, isopropyl alcohol, and lauryl alcohol. Examples of the fatty acid ester may include isopropyl myristate, isopropyl palmitate, and decyl oleate.

Examples of the monohydric alcohol may include isostearyl alcohol, octyldodecanol, and hexyldecanol.

The amount of the liquid solubilizing aid in the medicated layer is preferably 2 to 25% by weight, more preferably 5 to 20% by weight, and particularly preferably 10 to 15% by weight. If the amount of the liquid solubilizing aid in the medicated layer is small, the effect of enhancing the dissolvability and diffusibility of midodrine in the medicated layer becomes low, and the transdermal absorbability of midodrine becomes insufficient. If the amount of the liquid solubilizing aid in the medicated layer is large, the acrylic adhesive in the medicated layer is excessively plasticized, so that the adhesive remains on the skin when the patch is removed from the skin.

The solubility of midodrine in the liquid solubilizing aid at 25°C is preferably 0.01 to 1 and more preferably 0.01 to 0.5. If the solubility of midodrine in the liquid solubilizing aid at 25°C is low, the effect of enhancing the transdermal absorption of midodrine may not be obtained. If the solubility of midodrine in the liquid solubilizing aid at 25°C is high, the dissolvability of midodrine in the medicated layer becomes excessively high. Therefore, midodrine is easily supersaturated in the medicated layer, and the storage stability of midodrine may deteriorate. The solubility of midodrine in the liquid solubilizing aid at 25°C is the number of grams of midodrine in 100 g of the liquid solubilizing aid in a saturated solution obtained by dissolving midodrine in the liquid solubilizing aid.

A plasticizer, an absorption promoter, a stabilizer, a filler, etc. may be added to the medicated layer, so long as the effects of the present invention are not impaired.

The plasticizer is compatible with the acrylic adhesive and is used for the purpose of improving the adhesion of the medicated layer and of enhancing the diffusion rate of midodrine in the medicated layer. Examples of such a plasticizer may include saturated hydrocarbons such as liquid paraffin and squalane. The amount of the plasticizer in the medicated layer is preferably 0.05 to 15% by weight.

The absorption promoter acts on the skin and is used in order to improve the transdermal permeability of midodrine and for the purpose of softening the horny layer or enhancing hydration of the horny layer. Examples of such an absorption promoter may include surfactants such as polysorbate, lauric acid diethanolamide, lauroylsarcosine, polyoxyethylene alkyl ethers, and polyoxyethylene alkyl amines. The amount of the absorption promoter in the medicated layer is preferably 0.05 to 10% by weight.

The stabilizer is used for the purpose of suppressing the oxidation and decomposition of midodrine. Examples of the stabilizer may include: antioxidants such as butylhydroxytoluene and sorbic acid; cyclodextrin; and ethylenediaminetetraacetic acid. The amount of the stabilizer in the medicated layer is preferably 0.05 to 10% by weight.

The filler is used in order to control the adhesion of the patch and the transdermal absorbability of midodrine. Examples of the filler may include: organic metal salts such as calcium carbonate and magnesium stearate; inorganic fillers such as silicic acid anhydride and titanium oxide; lactose; cellulose derivatives such as crystalline cellulose, ethyl cellulose, and low substituted hydroxypropyl cellulose; and macromolecules obtained from monomers such as vinylpyrrolidone, (meth)acrylic acid, and derivatives of (meth)acrylic acid. The amount of the filler in the medicated layer is preferably 1 to 15% by weight.

The thickness of the medicated layer in the patch is preferably 10 to 250 μm, more preferably 20 to 200 μm, and particularly preferably 40 to 100 μm. If the thickness of the medicated layer is small, the medicated layer may not be capable of containing midodrine in an amount necessary to obtain the desired blood level of DMAE, or the adhesion to the skin surface becomes low, so that the patch applied to the skin may be peeled off. If the thickness of the medicated layer is large, the medicated layer is likely to bulge out of the patch during storage or after the patch is applied, or the application feeling of the patch applied to the skin may deteriorate.

Preferably, for the purpose of preventing loss of midodrine in the medicated layer in the patch of the present invention and of protecting the medicated layer, release liner is releasably stacked on and integrated with the surface of the medicated layer in the patch.

Examples of the release liner may include paper and resin films formed of polyethylene terephthalate, polyethylene, polypropylene, polyvinyl chloride, and polyvinylidene chloride. Preferably, the release liner has been subjected to release treatment on the surface facing the medicated layer. The release liner may include a single layer or a plurality of layers.

For the purpose of improving the barrier properties of the release liner, an aluminum foil or an aluminum vapor-deposition layer may be provided on the release liner. When the release liner is formed of paper, the release liner may be impregnated with a resin such as polyvinyl alcohol, for the purpose of improving the barrier properties of the release liner.

A method of producing the patch of the present invention will next be described. The type of method of producing the patch is not particularly limited. For example, when a solvent coating process is used, the following method can be used. Midodrine, the acrylic adhesive, and optionally added additives are added to a solvent such as ethyl acetate, and the mixture is stirred until uniform to obtain a medicated layer solution. The medicated layer solution is uniformly applied to one surface of the backing using a coater and then dried to stack and integrate a medicated layer on the one surface of the backing. If necessary, release liner is stacked on and integrated with the medicated layer such that the surface of the release liner that has been subjected to release treatment faces the medicated layer. In addition, the following method can be used. The medicated layer solution is applied to the surface of release liner that has been subjected to release treatment using the above coating process and then dried to form a medicated layer on the release liner. Then the backing is stacked on and integrated with the medicated layer.

Advantageous Effects of Invention

In the patch of the present invention, midodrine is contained in the prescribed acrylic adhesive. The dissolvability of midodrine in the medicated layer is appropriately suppressed, and the release of midodrine from the medicated layer to the skin is facilitated. Therefore, high transdermal absorbability of midodrine is achieved.

In addition, in the medicated layer, part of midodrine is not dissolved in the medicated layer and is stably present in the acrylic adhesive. Therefore, as the amount of midodrine contained in the acrylic adhesive decreases, the midodrine present in an undissolved state in the medicated layer is rapidly dissolved and diffused in the medicated layer, so that the patch of the present invention allows the transdermal absorbability of midodrine to be stably maintained for a long period of time.

Brief Description of Drawings

FIG. 1 is a graph showing the relation between the amount of midodrine in a medicated layer and the haze value of the medicated layer.

FIG. 2 is a graph showing the results of a midodrine release test performed after a patch in Example 26 is stored at room temperature for one month or at 60°C for one month.

FIG. 3 is a graph showing the results of a midodrine release test performed after a patch in Example 27 is stored at room temperature for one month or at 60°C for one month.

FIG. 4 is a graph showing the results of a midodrine release test performed immediately after the production of a patch in Example 28 or after the patch is store at 60°C for one month.

FIG. 5 is a graph showing the results of a midodrine release test performed immediately after the production of a patch in Example 29 or after the patch is store at 60°C for one month.

FIG. 6 is a graph showing the results of a midodrine release test performed after the patch in Comparative Example 13 is stored at 4°C for one month or at 60°C for one month.

FIG. 7 is a vertical cross-sectional view showing the patch of the present invention.

Description of Embodiments

Examples of the present invention will next be described, but the present invention is not limited to these Examples.

First, the dissolvability and solubility of midodrine in each of alkyl acrylates, alkyl methacrylates, acrylic acid, and 1-vinyl-2-pyrrolidone were evaluated by the following procedures, and the results are shown in TABLE 1.

(Dissolvability of midodrine)

One hundred parts by weight of a monomer shown in TABLE 1 and 3 parts by weight of midodrine were sufficiently mixed to prepare a midodrine solution, and the dissolved state of midodrine in the midodrine solution was visually observed. The midodrine solution was stored at a temperature of 25°C for 7 days, and then the dissolved state of midodrine was again visually observed. The dissolvability of midodrine was evaluated according to the following criteria. In addition, the solubility of midodrine in each of the monomers shown in TABLE 1 at 25°C was measured using the same procedure as that described later except that the monomers shown in TABLE 1 were used instead of compounds shown in TABLE 2.

High: No deposition of midodrine was found in any of the midodrine solution immediately after preparation and the midodrine solution after storage for 7 days.

Middle: Deposition of midodrine was found in the solution immediately after preparation, but no deposition of midodrine was found in the midodrine solution after storage for 7 days.

Low: Deposition of midodrine was found in the midodrine solution immediately after preparation and also in the midodrine solution after storage for 7 days.

Next, the storage stability and solubility of midodrine in each of saturated aliphatic monohydric alcohols, fatty acid esters, and additives used for patches were evaluated, and the results are shown in TABLE 2.

(Storage stability of midodrine)

Three parts by weight of midodrine and 100 parts by weight of a compound shown in TABLE 2 were mixed well, and the mixture was placed in a sealed container and stored under the temperature condition of 60°C for 20 days. Then the color of the mixture after storage was visually observed. When the color was found unchanged compared with that before the storage, the mixture was evaluated as "excellent." When discoloration was found, the mixture was evaluated as "bad."

The weight $W_5$ (g) of non-decomposed midodrine remaining in the mixture after storage was quantified by HPLC, and the ratio (% by weight) of remaining midodrine to the amount $W_6$ (g) of midodrine added was computed using the following formula (1).

$$\text{Ratio (\% by weight) of remaining midodrine}$$

$$= 100 \times (W_5/W_6) \qquad (1)$$

(Solubility of midodrine)

A midodrine solution composed of 50 parts by weight of midodrine and 950 parts by weight of a compound shown in TABLE 2 and used as a solvent was prepared. Then the midodrine solution was maintained at 50°C for 15 minutes and subjected to ultrasonic shaking in a water bath at 25°C for 10 minutes. The resultant midodrine solution was transferred to a centrifuge tube and left to stand at 25°C for 2 hours. Then the midodrine solution was centrifuged at a rotation speed of 3,000 rpm using a centrifuge, and the presence or absence of crystals of midodrine on the bottom of the centrifuge tube was observed. When no crystals of midodrine were deposited, the same procedure was repeated with the midodrine-to-compound weight ratio increased up to 200:800 to thereby prepare a saturated midodrine solution.

A certain amount of the supernatant of the saturated midodrine solution after centrifugation was collected, and the collected supernatant was subjected to HPLC measurement to quantify the weight $W_7$ (g) of midodrine dissolved in the saturated midodrine solution. Then the solubility (g) at 25°C of midodrine in the compound used as the solvent was computed using the following formula (2).

$$\text{Solubility (g) of midodrine} = 100 \times (W_7/W_8) \qquad (2)$$

($W_8$: weight (g) of compound used as solvent in saturated midodrine solution)

When the amount of midodrine in the saturated midodrine solution was equal to or less than the detection limit of HPLC, "N.D. (Not Detected)" was entered in TABLE 2 as the result of the HPLC measurement for this compound.

Next, acrylic adhesives A to L and N and a rubber-based adhesive M used as the adhesives of patches were prepared according to the following procedure, and then patches in Examples 1 to 29 and Comparative Examples 1 to 13 were produced.

(Preparation of acrylic adhesive A)

A separable flask was charged with a reaction mixture composed of 50 parts by weight of ethyl acrylate, 40 parts by weight of octyl acrylate, 10 parts by weight of 1-vinyl-2-pyrrolidone, and 120 parts by weight of ethyl acetate, and the atmosphere in the separable flask was replaced with a nitrogen atmosphere at 80°C. Then the reaction mixture was polymerized while a polymerization initiator solution prepared by dissolving 1 part by weight of lauroyl peroxide in 50 parts by weight of ethyl acetate was added to the reaction mixture over 24 hours. After the polymerization, ethyl acetate was further added to obtain an acrylic adhesive solution A containing 35% by weight of acrylic adhesive A.

(Preparation of acrylic adhesive B)

A separable flask was charged with a reaction mixture composed of 50 parts by weight of ethyl acrylate, 35 parts by weight of 2-ethylhexyl acrylate, 15 parts by weight of 1-vinyl-2-pyrrolidone, and 120 parts by weight of ethyl acetate, and the atmosphere in the separable flask was replaced with a nitrogen atmosphere at 80°C. Then the reaction mixture was polymerized while a polymerization initiator solution prepared by dissolving 0.3 parts by weight of azobisisobutyronitrile in 50 parts by weight of ethyl acetate was added to the reaction mixture over 18 hours. After the polymerization, ethyl acetate was further added to obtain an acrylic adhesive solution B containing 35% by weight of acrylic adhesive B.

(Preparation of acrylic adhesive C)

A separable flask was charged with a reaction mixture composed of 70 parts by weight of ethyl acrylate, 25 parts by weight of 2-ethylhexyl acrylate, 5 parts by weight of 1-vinyl-2-pyrrolidone, and 120 parts by weight of ethyl acetate, and the atmosphere in the separable flask was replaced with a nitrogen atmosphere at 80°C. Then the reaction mixture was polymerized while a polymerization initiator solution prepared by dissolving 1 part by weight of lauroyl peroxide in 50 parts by weight of ethyl acetate was added to the reaction mixture over 18 hours. After the polymerization, ethyl acetate was further added to obtain an acrylic adhesive solution C containing 35% by weight of acrylic adhesive C.

(Preparation of acrylic adhesive D)

A separable flask was charged with a reaction mixture composed of 30 parts by weight of ethyl acrylate, 40 parts by weight of 2-ethylhexyl acrylate, 30 parts by weight of 1-vinyl-2-pyrrolidone, and 120 parts by weight of ethyl acetate, and the atmosphere in the separable flask was replaced with a nitrogen atmosphere at 80°C. Then the reaction mixture was polymerized while a polymerization initiator solution prepared by dissolving 1 part by weight of lauroyl peroxide in 50 parts by weight of ethyl acetate was added to the reaction mixture over 18 hours. After the polymerization, ethyl acetate was further added to obtain an acrylic adhesive solution D containing 35% by weight of acrylic adhesive D.

(Preparation of acrylic adhesive E)

A separable flask was charged with a reaction mixture composed of 58 parts by weight of ethyl acrylate, 40 parts by weight of octyl acrylate, 2 parts by weight of 1-vinyl-2-pyrrolidone and 120 parts by weight of ethyl acetate, and the atmosphere in the separable flask was replaced with a nitrogen atmosphere at 80°C. Then the reaction mixture was polymerized while a polymerization initiator solution prepared by dissolving 1 part by weight of lauroyl peroxide in 50 parts by weight of ethyl acetate was added to the reaction mixture over 24 hours. After the polymerization, ethyl acetate was further added to obtain an acrylic adhesive solution E containing 35% by weight of acrylic adhesive E.

(Preparation of acrylic adhesive F)

A separable flask was charged with a reaction mixture composed of 25 parts by weight of ethyl acrylate, 70 parts by weight of 2-ethylhexyl acrylate, 5 parts by weight of 1-vinyl-2-pyrrolidone, and 120 parts by weight of ethyl acetate, and the atmosphere in the separable flask was replaced with a nitrogen atmosphere at 80°C. Then the reaction mixture was polymerized while a polymerization initiator solution prepared by dissolving 1 part by weight of lauroyl peroxide in 50 parts by weight of ethyl acetate was added to the reaction mixture over 18 hours. After the polymerization, ethyl acetate was further added to obtain an acrylic adhesive solution F containing 35% by weight of acrylic adhesive F.

(Preparation of acrylic adhesive G)

A separable flask was charged with a reaction mixture composed of 75 parts by weight of 2-ethylhexyl acrylate, 25 parts by weight of 1-vinyl-2-pyrrolidone, and 50 parts by weight of ethyl acetate, and the atmosphere in the separable flask was replaced with a nitrogen atmosphere at 80°C. Then the reaction mixture was polymerized while a polymerization initiator solution prepared by dissolving 0.5 parts by weight of azobisisobutyronitrile in 50 parts by weight of ethyl acetate was added to the reaction mixture over 18 hours. After the polymerization, ethyl acetate was further added to obtain an acrylic adhesive solution G containing 35% by weight of acrylic adhesive G.

(Preparation of acrylic adhesive H)

A separable flask was charged with a reaction mixture composed of 50 parts by weight of ethyl acrylate, 35 parts by weight of 2-ethylhexyl acrylate, 15 parts by weight of dodecyl methacrylate, and 50 parts by weight of ethyl acetate, and the atmosphere in the separable flask was replaced with a nitrogen atmosphere at 80°C. Then the reaction mixture was polymerized while a polymerization initiator solution prepared by dissolving 1 part by weight of benzoyl peroxide in 100 parts by weight of ethyl acetate was added to the reaction mixture over 18 hours. After the polymerization, ethyl acetate was further added to obtain an acrylic adhesive solution H containing 35% by weight of acrylic adhesive H.

(Preparation of acrylic adhesive I)

A separable flask was charged with a reaction mixture composed of 75 parts by weight of 2-ethylhexyl methacrylate, 25 parts by weight of 1-vinyl-2-pyrrolidone, and 50 parts by weight of ethyl acetate, and the atmosphere in the separable flask was replaced with a nitrogen atmosphere at 80°C. Then the reaction mixture was polymerized while a polymerization initiator solution prepared by dissolving 0.5 parts by weight of azobisisobutyronitrile in 50 parts by weight of ethyl acetate was further added to obtain an acrylic adhesive solution I containing 35% by weight of acrylic adhesive I.

(Preparation of acrylic adhesive J)

A separable flask was charged with a reaction mixture composed of 15 parts by weight of dodecyl methacrylate, 85 parts by weight of 2-ethylhexyl methacrylate, and 50 parts by weight of ethyl acetate, and the atmosphere in the separable flask was replaced with a nitrogen atmosphere at 80°C. Then the reaction mixture was polymerized while a polymerization initiator solution prepared by dissolving 0.5 parts by weight of azobisisobutyronitrile in 50 parts by weight of ethyl acetate was added to the reaction mixture over 18 hours. After the polymerization, ethyl acetate was further added to obtain an acrylic adhesive solution J containing 35% by weight of acrylic adhesive J.

(Preparation of acrylic adhesive K)

A separable flask was charged with a reaction mixture composed of 70 parts by weight of ethyl acrylate, 25 parts by weight of 2-ethylhexyl acrylate, 5 parts by weight of acrylic acid, and 80 parts by weight of ethyl acetate, and the atmosphere in the separable flask was replaced with a nitrogen atmosphere at 80°C. Then the reaction mixture was polymerized while a polymerization initiator solution prepared by dissolving 1 part by weight of benzoyl peroxide in 100 parts by weight of ethyl acetate was added to the reaction mixture over 18 hours. After the polymerization, ethyl acetate was further added to obtain an acrylic adhesive solution K containing 32% by weight of acrylic adhesive K.

(Preparation of acrylic adhesive L)

A separable flask was charged with a reaction mixture composed of 95 parts by weight of 2-ethylhexyl acrylate, 5 parts by weight of acrylic acid, and 50 parts by weight of ethyl acetate, and the atmosphere in the separable flask was replaced with a nitrogen atmosphere at 80°C. Then the reaction mixture was polymerized while a polymerization initiator solution prepared by dissolving 0.5 parts by weight of azobisisobutyronitrile in 50 parts by weight of ethyl acetate was added to the reaction mixture over 18 hours. After the polymerization, ethyl acetate was further added to obtain an acrylic adhesive solution L containing 35% by weight of acrylic adhesive L.

(Preparation of rubber-based adhesive M)

A separable flask was charged with 40 parts by weight of polyisobutylene A (product name: "Oppanol B80," manufactured by BASF), 30 parts by weight of polyisobutylene B (product name: "Himol 5H," manufactured by Nippon Petrochemicals Co., Ltd.), 30 parts by weight of polyisobutylene C (product name: "Nisseki Polybutene HV-300F," manufactured by Nippon Petrochemicals Co., Ltd.), and 300 parts by weight of toluene. The atmosphere in the separable flask was replaced with a nitrogen atmosphere, and the mixture was uniformly mixed to thereby obtain a rubber-based adhesive solution M containing 25% by weight of a polyisobutylene adhesive.

(Preparation of acrylic adhesive N)

A 40L polymerization apparatus was charged with a reaction mixture composed of 13 parts by weight of dodecyl methacrylate, 78 parts by weight of 2-ethylhexyl methacrylate, 9 parts by weight of 2-ethylhexyl acrylate, and 50 parts by weight of ethyl acetate, and the atmosphere in the polymerization apparatus was replaced with a nitrogen atmosphere at 80°C. Then the reaction mixture was polymerized while a polymerization initiator solution prepared by dissolving 1 part by weight of benzoyl peroxide in 100 parts by weight of cyclohexane was added to the reaction mixture over 24 hours. After the polymerization, ethyl acetate was further added to obtain an acrylic adhesive N solution containing 35% by weight of acrylic adhesive N.

In TABLEs 3 to 6, octyl acrylate is denoted as "OA," 2-ethylhexyl acrylate is denoted as "EHA," ethyl acrylate is denoted as "EA," dodecyl methacrylate is denoted as "DM," 2-ethylhexyl methacrylate is denoted as "EHM," 1-vinyl-2-pyrrolidone is denoted as "VP," and acrylic acid is denoted as "AA." In liquid solubilizing aid rows, the solubility at 25°C of midodrine in each liquid solubilizing aid was placed after the name of the liquid solubilizing aid.

(Examples 1 to 25 and Comparative Examples 1 to 12)

Midodrine, midodrine hydrochloride, an acrylic adhesive solution, the rubber-based adhesive solution, a liquid solubilizing aid, and an additive were mixed such that the weight composition ratios of midodrine, midodrine hydrochloride, the acrylic adhesive, the rubber-based adhesive, the liquid solubilizing aid, and the additive in medicated layers were as shown in TABLEs 3 to 5. Ethyl acetate or toluene was added such that the concentration of solids was 22 to 28% by weight, and then the mixture was mixed until uniform to prepare a medicated layer solution.

Next, a 38 μm-thick polyethylene terephthalate film subjected to silicon release treatment was prepared. The above medicated layer solution was applied to the silicon release-treated surface of the polyethylene terephthalate film and dried at 60°C for 30 minutes to produce a stacked body in which a medicated layer having a thickness shown in TABLEs 3 to 5 was formed on the silicon release-treated surface of the polyethylene terephthalate film.

Then a backing formed from a 25 μm-thick polyethylene terephthalate film was prepared. The above-produced stacked body was overlaid on the backing such that the medicated layer of the stacked body faced one surface of the backing, and the medicated layer was thereby stacked on and integrated with the backing to produce a patch.

Medicated layers having the same composition as that of the above produced patches were produced using the same procedure as described above for each of the Examples and Comparative Examples, in order to measure the haze values of the medicated layers. Polyethylene terephthalate films having a thickness of 25 μm and a haze value of 2 % were affixed to the both surfaces of each medicated layer, and the resultant medicated layer was cut into a flat rectangular shape of 3.5 cm length × 4.5 cm width to prepare a test piece for haze value measurement.

The amount of undissolved midodrine in each medicated layer was measured immediately after production and after storage at 40°C for one month using the above-described procedure. The percentage of the weight of undissolved midodrine in the medicated layer with respect to the total weight of midodrine contained in the medicated layer was given in TABLEs 3 to 5. For each patch, a permeability measurement test was performed using the following procedure immediately after production and after storage at 40°C for one month. The measurement results are shown in TABLEs 3 to 5.

(Permeability test)

For each of the patches produced in Examples 1 to 25 and Comparative Examples 1 to 12, a hairless mouse skin permeability test was performed immediately after production and after storage at 40°C for one month by the following method, in order to evaluate the absorbability of the drug into the skin.

A rounded square test piece with the side of 1.8 cm (application area: 3 cm²) was cut from a patch. Dorsal skin removed from a hairless mouse (male, 8 weeks old) was fixed on a Franz diffusion cell maintained at 37°C, and the test piece was applied to the upper end of the skin through the adhesive layer of the test piece. A physiological saline solution with its pH adjusted to 7.2 was used as a receptor solution, and the lower end of the skin was immersed in the receptor solution.

The receptor solution on the lower side of the skin was collected 4, 6, 22, and 24 hours after the test piece was applied to the skin, and the concentration of midodrine was measured by HPLC. The permeation amount of midodrine determined from the midodrine concentration and the amount of the receptor solution was computed at each time point, and the determined value was used as a cumulative skin permeation amount. When the permeation amounts of midodrine were computed after 6, 22, and 24 hours, since the receptor solution had been collected before these time points, the permeation amounts were corrected on the basis of the amount of the receptor solution collected.

(Examples 26 to 29 and Comparative Example 13)

Midodrine, an acrylic adhesive solution, and a liquid solubilizing aid were mixed such that the weight composition ratios of midodrine, the acrylic adhesive, and the liquid solubilizing aid in a medicated layer were as shown in TABLE 6. Ethyl acetate or toluene was added such that the concentration of solids was 22 to 28% by weight, and then the mixture was mixed until uniform to prepare a medicated layer solution.

Next, a 38 $\mu$m-thick polyethylene terephthalate film subjected to silicon release treatment was prepared. The above medicated layer solution was applied to the silicon release-treated surface of the polyethylene terephthalate film and dried at 60°C for 30 minutes to produce a stacked body in which a medicated layer having a thickness shown in TABLE 6 was formed on the silicon release-treated surface of the polyethylene terephthalate film.

Then, a backing prepared by stacking and integrating a 12 $\mu$m-thick polyethylene terephthalate film and a nonwoven polyester fiber fabric having a basis weight of 40 g/m$^2$ was used to produce a patch. More specifically, the backing was stacked on and integrated with the medicated layer such that the polyethylene terephthalate film of the backing faced the medicated layer.

Medicated layers having the same composition were produced using the same procedure as described above for each of the Examples and Comparative Example, in order to measure the haze values of the medicated layers. Polyethylene terephthalate films having a thickness of 25 $\mu$m and a haze value of 2 % were affixed to the both surfaces of each medicated layer, and the resultant medicated layer was cut into a flat rectangular shape of 3.5 cm length $\times$ 4.5 cm width to prepare a test piece for haze value measurement.

The amount of undissolved midodrine in each medicated layer was measured immediately after production and after storage at 40°C for one month using the above-described procedure. The percentage of the weight of undissolved midodrine in the medicated layer with respect to the total weight of midodrine contained in the medicated layer was given in TABLE 6. For each patch, a midodrine release test was performed using the following procedure. The results are shown in TABLE 6.

(Midodrine release test)

For each of the patches in Examples 26 to 29 and Comparative Example 13, a midodrine release test was performed by the following method immediately after production, after storage at 4°C for one month, or after storage at room temperature (25°C) for one month and after storage at 60°C for one month, as shown in TABLE 6, in order to evaluate the drug release rate from the medicated layer.

A flat square test piece having an area shown in TABLE 6 was cut from each patch. The four corners of the test piece were formed as a convex arc shape. A stainless steel plate was prepared, and the test piece was affixed to the stainless steel plate using a double-sided adhesive tape such that one surface of the stainless steel plate faced the backing of the test piece. Then the polyethylene terephthalate film subjected to silicon release treatment was removed to expose the medicated layer. Five hundred mL of water used as a test solution was supplied to a release test apparatus and maintained at 37°C. The test piece was immersed in the test solution such that the medicated layer faced upward, and the test solution was stirred at a speed of 100 rpm with stirring vanes.

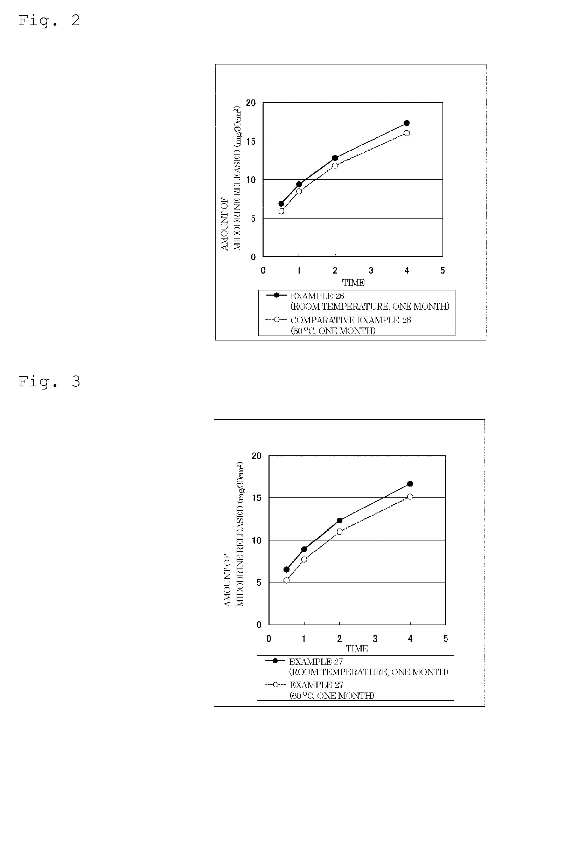

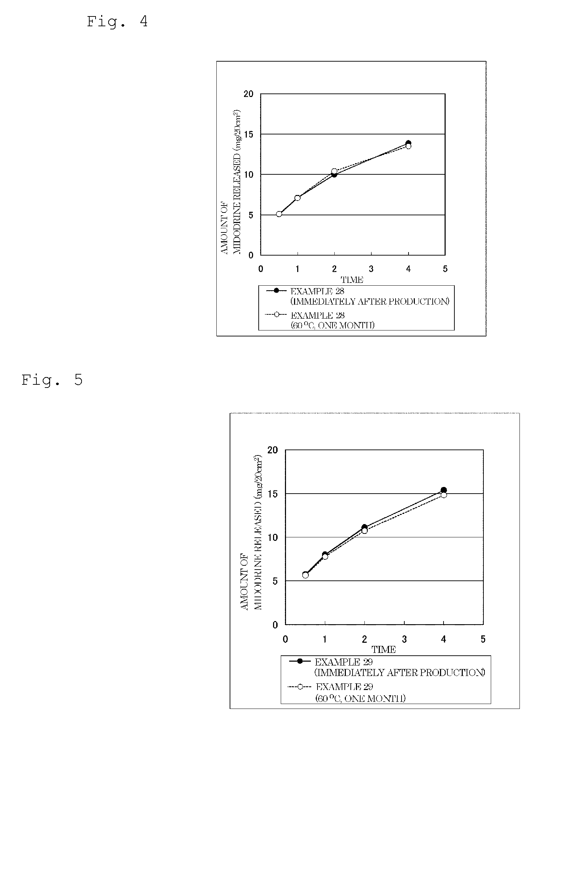

A certain amount of the test solution was collected 30, 60, 120, and 240 minutes after the test piece was immersed in the test solution, and the concentration of midodrine was measured by HPLC. The amount of released midodrine determined by the midodrine concentration and the amount of the test solution was computed at each time point. Three test pieces were prepared for each patch, and the arithmetic mean of the amounts of midodrine released from the respective test pieces was used as the amount of release. When the amounts of release were computed, considering the amount of the test solution which had been collected in advance, the amounts of the collected test solution were corrected. The results are shown in FIGs. 2 to 6.

[Table 1]

| MONOMER | DISSOLVABILITY OF MIDODRINE | SOLUBILITY OF MIDODRINE |
|---|---|---|
| DODECYL METHACRYLATE | LOW | 0.02 |
| 2-ETHYLHEXYL METHACRYLATE | LOW | 0.02 |
| BUTYL METHACRYLATE | LOW | 0.10 |

(continued)

| MONOMER | DISSOLVABILITY OF MIDODRINE | SOLUBILITY OF MIDODRINE |
|---|---|---|
| 2-ETHYLHEXYL ACRYLATE | LOW | 0.03 |
| ETHYL ACRYLATE | LOW | 0.25 |
| BUTYL ACRYLATE | LOW | 0.11 |
| ACRYLIC ACID | HIGH | >3.0 |
| 1-VINYL-2-PYRROLIDONE | HIGH | >3.0 |

[Table 2]

| COMPOUND | STORAGE STABILITY OF MIDODRINE | | SOLUBILITY OF MIDODRINE |
|---|---|---|---|
| | DISCOLORATION TEST | RATIO OF REMAINING MIDODRINE (% BY WEIGHT) | |
| HEXYLDECANOL | EXCELLENT | 90.4 | 0.26 |
| OCTYLDODECANOL | EXCELLENT | 100.0 | 0.11 |
| ISOSTEARYL ALCOHOL | EXCELLENT | 99.4 | 0.16 |
| LAURYL ALCOHOL | BAD | 78.3 | 0.63 |
| OLEYL ALCOHOL | BAD | 29.8 | 0.40 |
| ISOPROPYL MYRISTATE | EXCELLENT | 99.3 | 0.02 |
| ISOPROPYL PALMITATE | EXCELLENT | 100.0 | 0.02 |
| OCTYLDODECYL MYRISTATE | EXCELLENT | 94.8 | 0.01 |
| HEXYL LAURATE | EXCELLENT | 96.3 | 0.02 |
| MEDIUM- CHAIN FATTY ACID TRIGLYCERIDE | EXCELLENT | 82.0 | 0.05 |
| TRIETHYL CITRATE | EXCELLENT | 29.0 | 1.59 |
| TRIACETIN | EXCELLENT | 7.7 | 0.70 |
| MONOETHANOLAMINE | BAD | 63.5 | 16.2 |
| LIQUID PARAFFIN | EXCELLENT | 100.0 | N.D. |
| SQUALANE | EXCELLENT | 100.0 | N.D. |
| BUTYLENE GLYCOL | EXCELLENT | 96.6 | 7.72 |
| PROPYLENE GLYCOL | EXCELLENT | 90.1 | 15.67 |
| OCTANEDIOL | BAD | 48.0 | 1.78 |
| CONCENTRATED GLYCERIN | BAD | 58.2 | 4.07 |
| POLYETHYLENE GLYCOL 400 | BAD | 6.0 | 9.23 |
| POLYOXYETHYLENE (10) HYDROGENATED CASTOR OIL | BAD | 52.0 | 0.70 |
| ISOSTEARIC ACID | BAD | 4.8 | 15.17 |

[Table 3]

| COMPOSITION | | | EXAMPLE 1 | EXAMPLE 2 | EXAMPLE 3 | EXAMPLE 4 | EXAMPLE 5 | EXAMPLE 6 | EXAMPLE 7 | EXAMPLE 8 | EXAMPLE 9 | EXAMPLE 10 | EXAMPLE 11 | EXAMPLE 12 | EXAMPLE 13 | EXAMPLE 14 | EXAMPLE 15 | EXAMPLE 16 | EXAMPLE 17 | EXAMPLE 18 | EXAMPLE 19 |
|---|---|---|---|---|---|---|---|---|---|---|---|---|---|---|---|---|---|---|---|---|---|
| MIDODRINE (% BY WEIGHT) | | | 10 | 10 | 10 | 10 | 10 | 10 | 25 | 20 | 15 | 10 | 5 | 3 | 3 | 3 | 15 | 15 | 15 | 15 | 15 |
| MIDODRINE HYDROCHLORIDE (% BY WEIGHT) | | | — | — | — | — | — | — | — | — | — | — | — | — | — | — | — | — | — | — | — |
| ACRYLIC ADHESIVE (% BY WEIGHT) | A | OA·EA·VP=40·50·10 | — | — | — | — | — | — | 65 | 70 | 75 | 80 | 85 | 87 | 87 | — | 85 | 83 | 80 | 75 | 75 |
| | B | EHA·EA·VP=35·50·15 | 80 | — | — | — | — | — | — | — | — | — | — | — | — | — | — | — | — | — | — |
| | C | EHA·EA·VP=25·70·5 | — | 80 | — | — | — | — | — | — | — | — | — | — | — | — | — | — | — | — | — |
| | D | EHA·EA·VP=40·30·30 | — | — | 80 | — | — | — | — | — | — | — | — | — | — | 87 | — | — | — | — | — |
| | E | OA·EA·VP=40·58·2 | — | — | — | 80 | — | — | — | — | — | — | — | — | — | — | — | — | — | — | — |
| | F | EHA·EA·VP=70·25·5 | — | — | — | — | 80 | — | — | — | — | — | — | — | — | — | — | — | — | — | — |
| | G | EHA·VP=75·25 | — | — | — | — | — | 80 | — | — | — | — | — | — | — | — | — | — | — | — | — |
| | H | EHA·EA·DM=35·50·15 | — | — | — | — | — | — | — | — | — | — | — | — | — | — | — | — | — | — | — |
| | I | EHM·VP=75·25 | — | — | — | — | — | — | — | — | — | — | — | — | — | — | — | — | — | — | — |
| | J | DM·EHM=15·85 | — | — | — | — | — | — | — | — | — | — | — | — | — | — | — | — | — | — | — |
| | K | EHA·EA·AA=25·70·5 | — | — | — | — | — | — | — | — | — | — | — | — | — | — | — | — | — | — | — |
| | L | EHA·AA=95·5 | — | — | — | — | — | — | — | — | — | — | — | — | — | — | — | — | — | — | — |
| RUBBER BASED ADHESIVE (% BY WEIGHT) | M | (POLYISOBUTYLENE ADHESIVE) | — | — | — | — | — | — | — | — | — | — | — | — | — | — | — | — | — | — | — |
| LIQUID SOLUBILIZING AID (% BY WEIGHT) | | OCTYLDODECANOL (0.11) | — | — | — | — | — | — | — | — | — | — | — | — | 10 | — | — | 2 | 5 | 10 | — |
| | | ISOSTEARYL ALCOHOL (0.16) | — | — | — | — | — | — | — | — | — | — | — | — | — | — | — | — | — | — | 10 |
| | | HEXYLDECANOL (0.26) | — | — | — | — | — | — | — | — | — | — | — | — | — | — | — | — | — | — | — |
| | | LAURYL ALCOHOL (0.63) | — | — | — | — | — | — | — | — | — | — | — | — | — | — | — | — | — | — | — |
| | | ISOPROPYL MYRISTATE (0.02) | 10 | 10 | 10 | 10 | 10 | 10 | 10 | 10 | 10 | 10 | 10 | 10 | — | 10 | — | — | — | — | — |
| | | ISOPROPYL PALMITATE (0.02) | — | — | — | — | — | — | — | — | — | — | — | — | — | — | — | — | — | — | — |
| ADDITIVE (% BY WEIGHT) | | ISOSTEARIC ACID | — | — | — | — | — | — | — | — | — | — | — | — | — | — | — | — | — | — | — |
| THICKNESS OF MEDICATED LAYER ($\mu$m) | | | 70 | 70 | 70 | 70 | 70 | 70 | 50 | 50 | 50 | 70 | 70 | 70 | 70 | 70 | 50 | 50 | 50 | 50 | 50 |
| AMOUNT OF UNDISSOLVED MIDODRINE (% BY WEIGHT) | | IMMEDIATELY AFTER PRODUCTION | 83.0 | 90.3 | 85.3 | 93.5 | 96.0 | 86.9 | 95.3 | 93.7 | 91.0 | 85.5 | 68.9 | 45.7 | 42.7 | 32.5 | 90.0 | 89.7 | 89.8 | 90.4 | 89.2 |
| | | AFTER ONE MONTH AT 40°C | 82.1 | 89.5 | 84.2 | 93.1 | 94.9 | 85.4 | 94.2 | 93.0 | 90.5 | 85.1 | 68.0 | 45.1 | 41.9 | 21.8 | 89.6 | 89.3 | 89.2 | 90.1 | 88.6 |
| PERMEABILITY TEST ($\mu$g/cm²·24 HOURS) | | IMMEDIATELY AFTER PRODUCTION | 485 | 416 | 423 | 382 | 287 | 336 | 693 | 630 | 571 | 403 | 228 | 186 | 171 | 147 | 244 | 281 | 349 | 485 | 392 |
| | | AFTER ONE MONTH AT 40°C | 496 | 402 | 425 | 354 | 283 | 300 | 684 | 582 | 547 | 389 | 230 | 191 | 163 | 136 | 227 | 272 | 330 | 481 | 364 |

[Table 4]

| | | | EXAMPLE 20 | EXAMPLE 21 | EXAMPLE 22 | EXAMPLE 23 | EXAMPLE 24 | EXAMPLE 25 |
|---|---|---|---|---|---|---|---|---|
| MIDODRINE (% BY WEIGHT) | | | 15 | 10 | 10 | 10 | 5 | 3 |
| MIDODRINE HYDROCHLORIDE (% BY WEIGHT) | | | — | — | — | — | — | — |
| COMPOSITION | ACRYLIC ADHESIVE (% BY WEIGHT) | A OA·EA·VP=40·50·10 | 75 | 90 | 80 | 80 | 85 | 72 |
| | | B EHA·EA·VP=35·50·15 | — | — | — | — | — | — |
| | | C EHA·EA·VP=25·70·5 | — | — | — | — | — | — |
| | | D EHA·EA·VP=40·30·30 | — | — | — | — | — | — |
| | | E OA·EA·VP=40·58·2 | — | — | — | — | — | — |
| | | F EHA·EA·VP=70·25·5 | — | — | — | — | — | — |
| | | G EHA·VP=75·25 | — | — | — | — | — | — |
| | | H EHA·EA·DM=35·50·15 | — | — | — | — | — | — |
| | | I EHM·VP=75·25 | — | — | — | — | — | — |
| | | J DM·EHM=15·85 | — | — | — | — | — | — |
| | | K EHA·EA·AA=25·70·5 | — | — | — | — | — | — |
| | | L EHA·AA=95·5 | — | — | — | — | — | — |
| | RUBBER BASED ADHESIVE (% BY WEIGHT) | M (POLYISOBUTYLENE ADHESIVE) | — | — | — | — | — | — |
| | LIQUID SOLUBILIZING AID (% BY WEIGHT) | OCTYLDODECANOL (0.11) | — | — | — | 5 | — | — |
| | | ISOSTEARYL ALCOHOL (0.16) | — | — | 10 | — | — | — |
| | | HEXYLDECANOL (0.26) | — | — | — | — | — | 25 |
| | | LAURYL ALCOHOL (0.63) | — | — | — | — | — | — |
| | | ISOPROPYL MYRISTATE (0.02) | — | — | — | 5 | — | — |
| | | ISOPROPYL PALMITATE (0.02) | 10 | — | — | — | — | — |
| | ADDITIVE (% BY WEIGHT) | ISOSTEARIC ACID | — | — | — | — | 10 | — |
| THICKNESS OF MEDICATED LAYER ($\mu$m) | | | 50 | 70 | 70 | 70 | 70 | 70 |
| AMOUNT OF UNDISSOLVED MIDODRINE (% BY WEIGHT) | IMMEDIATELY AFTER PRODUCTION | | 90.9 | 84.2 | 84.8 | 84.8 | 35.6 | 52.4 |
| | AFTER ONE MONTH AT 40°C | | 90.7 | 83.8 | 81.3 | 84.5 | 14.1 | 50.9 |
| PERMEABILITY TEST ($\mu$g/cm$^2$ 24 HOURS) | IMMEDIATELY AFTER PRODUCTION | | 417 | 324 | 464 | 351 | 150 | 184 |
| | AFTER ONE MONTH AT 40°C | | 396 | 301 | 438 | 339 | 122 | 157 |

[Table 5]

| | | COMPARATIVE EXAMPLE 1 | COMPARATIVE EXAMPLE 2 | COMPARATIVE EXAMPLE 3 | COMPARATIVE EXAMPLE 4 | COMPARATIVE EXAMPLE 5 | COMPARATIVE EXAMPLE 6 | COMPARATIVE EXAMPLE 7 | COMPARATIVE EXAMPLE 8 | COMPARATIVE EXAMPLE 9 | COMPARATIVE EXAMPLE 10 | COMPARATIVE EXAMPLE 11 | COMPARATIVE EXAMPLE 12 |
|---|---|---|---|---|---|---|---|---|---|---|---|---|---|
| MIDODRINE (% BY WEIGHT) | | 10 | 10 | 10 | 10 | 10 | 10 | — | 1 | 2 | 1 | 1 | — |
| MIDODRINE HYDROCHLORIDE (% BY WEIGHT) | | — | — | — | — | — | — | 10 | — | — | — | — | 17 |
| ACRYLIC ADHESIVE (% BY WEIGHT) | A OA·EA·VP=40·50·10 | — | — | — | — | — | — | 80 | 89 | 88 | 89 | — | 73 |
| | B EHA·EA·VP=35·50·15 | — | — | — | — | — | — | — | — | — | — | — | — |
| | C EHA·EA·VP=25·70·5 | — | — | — | — | — | — | — | — | — | — | — | — |
| | D EHA·EA·VP=40·30·30 | — | — | — | — | — | — | — | — | — | — | 89 | — |
| | E OA·EA·VP=40·58·2 | — | — | — | — | — | — | — | — | — | — | — | — |
| | F EHA·EA·VP=70·25·5 | — | — | — | — | — | — | — | — | — | — | — | — |
| | G EHA·VP=75·25 | — | — | — | — | — | — | — | — | — | — | — | — |
| | H EHA·EA·DM=35·50·15 | 80 | — | — | — | — | — | — | — | — | — | — | — |
| | I EHM·VP=75·25 | — | 80 | — | — | — | — | — | — | — | — | — | — |
| | J DM·EHM=15·85 | — | — | 80 | — | — | — | — | — | — | — | — | — |
| | K EHA·EA·AA=25·70·5 | — | — | — | 80 | — | — | — | — | — | — | — | — |
| | L EHA·AA=95·5 | — | — | — | — | 80 | — | — | — | — | — | — | — |
| RUBBER BASED ADHESIVE (% BY WEIGHT) | M (POLYISOBUTYLENE ADHESIVE) | — | — | — | — | — | 80 | — | — | — | — | — | — |
| LIQUID SOLUBILIZING AID (% BY WEIGHT) | OCTYLDODECANOL (0.11) | — | — | — | — | — | — | — | — | 10 | 10 | — | 10 |
| | ISOSTEARYL ALCOHOL (0.16) | — | — | — | — | — | — | — | — | — | — | — | — |
| | HEXYLDECANOL (0.26) | — | — | — | — | — | — | — | — | — | — | — | — |
| | LAURYL ALCOHOL (0.63) | — | — | — | — | — | — | — | — | — | — | — | — |
| | ISOPROPYL MYRISTATE (0.02) | 10 | 10 | 10 | 10 | 10 | 10 | 10 | 10 | — | — | 10 | — |
| | ISOPROPYL PALMITATE (0.02) | — | — | — | — | — | — | — | — | — | — | — | — |
| ADDITIVE (% BY WEIGHT) | ISOSTEARIC ACID | — | — | — | — | — | — | — | — | — | — | — | — |
| THICKNESS OF MEDICATED LAYER ($\mu$m) | | 70 | 70 | 70 | 70 | 70 | 70 | 70 | 210 | 70 | 70 | 210 | 50 |
| AMOUNT OF UNDISSOLVED MIDODRINE (% BY WEIGHT) | IMMEDIATELY AFTER PRODUCTION | 90.3 | 87.9 | 96.0 | 70.8 | 74.8 | 96.8 | 96.7 | 0.0 | 16.6 | 0.0 | 0.0 | 98.3 |
| | AFTER ONE MONTH AT 40°C | 89.9 | 87.7 | 95.9 | 70.1 | 73.9 | 96.5 | 95.8 | 0.0 | 14.8 | 0.0 | 0.0 | 97.7 |
| PERMEABILITY TEST ($\mu$g/cm$^2$·24 HOURS) | IMMEDIATELY AFTER PRODUCTION | 303 | 138 | 122 | 134 | 91 | 61 | 42 | 125 | 111 | 51 | 90 | 51 |
| | AFTER ONE MONTH AT 40°C | 359 | 135 | 101 | 102 | 63 | 58 | 31 | 84 | 83 | 39 | 67 | 32 |

EP 2 659 886 A1

18

[Table 6]

| COMPOSITION | | Category | EXAMPLE 26 | EXAMPLE 27 | EXAMPLE 28 | EXAMPLE 29 | COMPARATIVE EXAMPLE 13 |
|---|---|---|---|---|---|---|---|
| MIDODRINE (% BY WEIGHT) | | | 10 | 10 | 15 | 15 | 10 |
| MIDODRINE HYDROCHLORIDE (% BY WEIGHT) | | | — | — | — | — | — |
| ACRYLIC ADHESIVE (% BY WEIGHT) | A | OA·EA·VP=40·50·10 | 80 | 80 | 75 | 75 | — |
| | B | EHA·EA·VP=35·50·15 | — | — | — | — | — |
| | C | EHA·EA·VP=25·70·5 | — | — | — | — | — |
| | D | EHA·EA·VP=40·30·30 | — | — | — | — | — |
| | E | OA·EA·VP=40·58·2 | — | — | — | — | — |
| | F | EHA·EA·VP=70·25·5 | — | — | — | — | — |
| | G | EHA·VP=75·25 | — | — | — | — | — |
| | H | EHA·EA·DM=35·50·15 | — | — | — | — | — |
| | I | EHM·VP=75·25 | — | — | — | — | — |
| | J | DM·EHM=15·85 | — | — | — | — | — |
| | K | EHA·EA·AA=25·70·5 | — | — | — | — | — |
| | L | EHA·AA=95·5 | — | — | — | — | — |
| | N | DM·EHM·EHA=13·78·9 | — | — | — | — | — |
| RUBBER BASED ADHESIVE (% BY WEIGHT) | M | (POLYISOBUTYLENE ADHESIVE) | — | — | — | — | 80 |
| LIQUID SOLUBILIZING AID (% BY WEIGHT) | | OCTYLDODECANOL (0.11) | — | — | 10 | — | — |
| | | ISOSTEARYL ALCOHOL (0.16) | 10 | — | — | — | 10 |
| | | HEXYLDECANOL (0.26) | — | — | — | — | — |
| | | LAURYL ALCOHOL (0.63) | — | — | — | — | — |
| | | ISOPROPYL MYRISTATE (0.02) | — | 10 | — | 10 | — |
| | | ISOPROPYL PALMITATE (0.02) | — | — | — | — | — |
| ADDITIVE (% BY WEIGHT) | | ISOSTEARIC ACID | — | — | — | — | — |
| THICKNESS OF MEDICATED LAYER 1 (μm) | | | 70 | 70 | 75 | 75 | 70 |
| AMOUNT OF UNDISSOLVED MIDODRINE (% BY WEIGHT) | | IMMEDIATELY AFTER PRODUCTION | 84.8 | 85.5 | 90.5 | 91.2 | 94.8 |
| | | AFTER ONE MONTH AT 40°C | 84.5 | 85.1 | 90.1 | 90.8 | 94.6 |
| AREA OF TEST PIECE (cm²) | | | 30 | 30 | 20 | 20 | 30 |
| MIDODRINE RELEASE TEST CONDITIONS | | STORAGE CONDITIONS 1 | AFTER ONE MONTH AT ROOM TEMPERATURE | AFTER ONE MONTH AT ROOM TEMPERATURE | IMMEDIATELY AFTER PRODUCTION | IMMEDIATELY AFTER PRODUCTION | AFTER ONE MONTH AT 4°C |
| | | STORAGE CONDITIONS 2 | AFTER ONE MONTH AT 60°C | AFTER ONE MONTH AT 60°C | AFTER ONE MONTH AT 60°C | AFTER ONE MONTH AT 60°C | AFTER ONE MONTH AT 60°C |

Industrial Applicability

The patch of the present invention is a midodrine transdermal absorption formulation. More specifically, the patch allows the transdermal absorbability of midodrine to be maintained for a long period of time.

Reference Signs List

L$_1$    first calibration line
L$_2$    second calibration line
C$_1$    saturating concentration
H$_1$    haze value

**Claims**

1.  A patch comprising: a backing; and a medicated layer stacked on one surface of the backing so as to be integrated with the backing, the medicated layer containing 3 to 40% by weight of midodrine and 60 to 97% by weight of an acrylic adhesive including a copolymer prepared by copolymerization of monomers including 60 to 98% by weight of an alkyl acrylate having an alkyl group with 1 to 12 carbon atoms and 2 to 40% by weight of 1-vinyl-2-pyrrolidone, wherein part of the midodrine is present in an undissolved state.

2.  The patch according to claim 1, wherein an amount of the midodrine present in the undissolved state in the medicated layer is 40 to 97% by weight based on the total amount of midodrine contained in the medicated layer.

3.  The patch according to claim 1, wherein the acrylic adhesive includes the copolymer containing 25 to 70% by weight of an alkyl acrylate component having an alkyl group with 1 to 3 carbon atoms, 25 to 70% by weight of an alkyl acrylate component having an alkyl group with 4 to 12 carbon atoms, and 2 to 30% by weight of a 1-vinyl-2-pyrrolidone component.

4.  The patch according to claim 1, wherein the acrylic adhesive includes the copolymer containing 25 to 70% by weight of an ethyl acrylate component, 25 to 70% by weight of an n-octyl acrylate component and/or a 2-ethylhexyl acrylate component, and 2 to 30% by weight of a 1-vinyl-2-pyrrolidone component.

5.  The patch according to claim 1, wherein the medicated layer includes a liquid solubilizing aid in an amount of 2 to 25% by weight, and the solubility of midodrine in the liquid solubilizing aid at 25°C is 0.01 to 1.

6.  The patch according to claim 5, wherein the liquid solubilizing aid is at least one compound selected from the group consisting of isopropyl myristate, isopropyl palmitate, octyldodecanol, hexyldecanol, and isostearyl alcohol.

Fig. 1

EP 2 659 886 A1

Fig. 2

Fig. 3

Fig. 4

Fig. 5

Fig. 6

Fig. 7

## INTERNATIONAL SEARCH REPORT

| International application No. |
| --- |
| PCT/JP2010/073783 |

**A. CLASSIFICATION OF SUBJECT MATTER**
*A61K31/165*(2006.01)i, *A61K9/70*(2006.01)i, *A61K47/10*(2006.01)i, *A61K47/14*(2006.01)i, *A61K47/32*(2006.01)i, *A61P13/02*(2006.01)i

According to International Patent Classification (IPC) or to both national classification and IPC

**B. FIELDS SEARCHED**

Minimum documentation searched (classification system followed by classification symbols)
A61K31/165, A61K9/70, A61K47/10, A61K47/14, A61K47/32, A61P13/02

Documentation searched other than minimum documentation to the extent that such documents are included in the fields searched
| Jitsuyo Shinan Koho | 1922–1996 | Jitsuyo Shinan Toroku Koho | 1996–2011 |
| Kokai Jitsuyo Shinan Koho | 1971–2011 | Toroku Jitsuyo Shinan Koho | 1994–2011 |

Electronic data base consulted during the international search (name of data base and, where practicable, search terms used)
CAplus/MEDLINE/EMBASE(STN), JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | JP 11-139968 A (Toa Eiyo Ltd.),<br>25 May 1999 (25.05.1999),<br>paragraphs [0027] to [0029]; comparative examples<br>(Family: none) | 1,2<br>5,6<br>3,4 |
| Y | WO 2009/113504 A1 (Sekisui Medical Co., Ltd.),<br>17 September 2009 (17.09.2009),<br>paragraphs [0028] to [0036]; examples 1 to 37<br>(Family: none) | 1-6 |
| Y | JP 2009-242303 A (Sekisui Medical Co., Ltd.),<br>22 October 2009 (22.10.2009),<br>paragraphs [0009], [0010], [0017], [0056];<br>tables 2, 3; comparative examples 5, 6<br>(Family: none) | 1-6 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>14 February, 2011 (14.02.11) | Date of mailing of the international search report<br>22 February, 2011 (22.02.11) |
| --- | --- |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

| | International application No. |
|---|---|
| | PCT/JP2010/073783 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 10-182445 A  (Sekisui Chemical Co., Ltd.), 07 July 1998 (07.07.1998), paragraphs [0019], [0043] to [0045], [0050]; table 2; examples 1 to 3 (Family: none) | 1-6 |
| Y | JP 2001-131062 A  (Yutoku Pharmaceutical Ind. Co., Ltd.), 15 May 2001 (15.05.2001), paragraphs [0004], [0017] to [0020] (Family: none) | 1-6 |
| Y | JP 2005-29541 A  (Shiseido Co., Ltd.), 03 February 2005 (03.02.2005), paragraph [0028] (Family: none) | 1-6 |
| Y | WO 2010/110320 A1  (Lintec Corp.), 30 September 2010 (30.09.2010), paragraph [0025] (Family: none) | 1-6 |
| Y | JP 2000-136134 A  (Eisai Co., Ltd.), 16 May 2000 (16.05.2000), paragraph [0013] & US 6576677 B1       & EP 1025858 A1 & WO 2000/012135 A1 | 1-6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001131062 A